# FASCICULE DE BREVET EUROPEEN

(11) **EP 0 925 106 B1**
(45) Date de publication et mention de la délivrance du brevet: **22.08.2001**
(21) Numéro de dépôt: 97926054.4
(22) Date de dépôt: 29.05.1997
(51) Int. Cl.: B01D 61/14, B01J 8/00, C07C 209/48, B01J 38/00

(54) **PROCEDE DE FILTRATION D'UN MELANGE REACTIONNEL TRIPHASIQUE**
VERFAHREN ZUR FILTRATION VON DREIPHASEN-REAKTIONSGEMISCHEN
METHOD FOR FILTERING A THREE-PHASED REACTION MIXTURE

(30) Priorité: 04.06.1996 FR 9607169
(43) Date de publication de la demande: 30.06.1999
(73) Titulaire: RHODIA POLYAMIDE INTERMEDIATES, 69190 Saint-Fons (FR)
(72) Inventeur: PERRONA, Philippe, F-01120 Montluel (FR); SEVER, Lionel, F-69007 Lyon (FR)
(74) Mandataire: Esson, Jean-Pierre
(86) Numéro de dépôt international: FR9700937
(87) Numéro de publication internationale: WO9746306

(56) Documents cités:
- EP-A- 0 052 719
- EP-A- 0 235 003
- EP-A- 0 599 180
- WO-A-91/16294
- DE-A- 3 245 318
- US-A- 2 166 183
- PATENT ABSTRACTS OF JAPAN vol. 017, no. 388 (C-1086), 21 Juillet 1993 & JP 05 068869 A (ASAHI CHEM IND CO LTD), 23 Mars 1993, & DATABASE WPI Section Ch, Week 9317 Derwent Publications Ltd., London, GB; Class E19, AN 93-136921
- PATENT ABSTRACTS OF JAPAN vol. 018, no. 134 (C-1176), 4 Mars 1994 & JP 05 317867 A (JAPAN ORGANO CO LTD), 3 Décembre 1993, & DATABASE WPI Section Ch, Week 9402 Derwent Publications Ltd., London, GB; Class D15, AN 94-011316

## Description

La présente invention concerne un procédé de filtration d'un mélange réactionnel triphasique, comportant une phase liquide, une phase catalytique solide non dissoute et une phase gazeuse.

Dans les procédés mettant en oeuvre un composé comportant au moins une fonction nitrile et un catalyseur non dissous de type nickel ou cobalt de Raney, il a été constaté qu'en l'absence de gaz, et plus particulièrement en l'absence d'hydrogène, les fonctions nitrile du milieu ont une influence néfaste sur l'activité du catalyseur.

Ainsi dans les procédé d'hydrogénation des nitriles en amines et encore plus particulièrement dans les procédés d'hydrogénation partielle d'un dinitrile en aminonitrile, la Demanderesse a mis en évidence que le catalyseur de type nickel ou cobalt de Raney a tendance à se désactiver en présence de fonctions nitrile, lorsque le milieu ne contient plus ou très peu d'hydrogène.

D'autre part; il s'avère que les catalyseurs métalliques déposés sur un support, qui sont utilisés aussi, notamment dans des procédés d'hydrogénation, décantent mal et sont difficilement filtrables.

La présente invention propose une solution à ces problèmes, consistant à filtrer tangentiellement sur un filtre à membrane au moins une partie d'un mélange réactionnel triphasique issu d'une réaction d'hydrogénation comportant une phase liquide dans laquelle se trouvent des fonctions nitrile, une phase gazeuse comprenant de l'hydrogène et une phase solide catalytique comprenant du nickel et/ou du cobalt de Raney ou un catalyseur métallique supporté, à recycler le catalyseur filtré tout en soutirant au moins une partie du filtrat contenant les produits de la réaction.

La circulation tangentielle, par rapport à la membrane et avec une vitesse élevée, du mélange à filtrer permet de réduire au maximum la quantité de solide retenue sur le filtre. Cela permet ainsi de minimiser les risques de désactivation du catalyseur. Elle permet de filtrer en continu tout en maintenant le catalyseur dans son milieu réactionnel.

Généralement la vitesse de circulation du mélange réactionnel sur la membrane est comprise entre 0,5 mètre/seconde et 20 mètres/seconde et de préférence entre 1 mètre/seconde et 10 mètres/seconde.

Les filtres à membrane utilisés pour la filtration tangentielle sont généralement constitués d'un support plan ou tubulaire et d'une membrane minérale ou organique, souvent appelée couche active, de quelques micromètres d'épaisseur.

Pour le procédé de l'invention, on utilisera de préférence des membranes minérales ayant généralement une meilleure tenue chimique et thermique au mélange réactionnel mis en oeuvre.

Le support et la couche active peuvent être réalisés dans le même matériau ou dans des matériaux différents. La couche active du filtre peut par exemple être en alpha-alumine, en oxyde de zirconium, en dioxyde de titane, en fibres de graphite. Le support peut aussi être en alumine, en graphite, en oxyde de zirconium, en dioxyde de titane.

La membrane est caractérisée également par son diamètre moyen de pores. Généralement ce diamètre moyen de pores varie entre 1 nanomètre et un micromètre.

Pour des questions de durée de vie et de régénérabilité de la membrane, on préfère utiliser dans le présent procédé des membranes d'ultrafiltration ayant un diamètre moyen de pores de 10 nanomètres à 100 nanomètres et un seuil de coupure (qui se définit par la masse moléculaire des composés arrêtés par la membrane et s'exprime en grammes/mole ou daltons) supérieur ou égal à 5 kilodaltons (kD) et de préférence supérieur ou égal à 100 kilodaltons.

La phase liquide du mélange réactionnel à filtrer comprend essentiellement au moins un composé à fonctions nitrile, tel que le dinitrile ou le nitrile de départ n'ayant pas été transformé, l'aminonitrile et/ou l'amine et/ou la diamine formés, un éventuel solvant qui peut être notamment l'eau et/ou un amide et/ou un alcool et/ou une amine et/ou de l'ammoniac. Les alcools qui sont le plus souvent utilisés sont les alcanols comme le méthanol, l'éthanol, le propanol-1, le propanol-2 et le butanol-1, les diols comme l'éthylèneglycol et le propylèneglycol, les polyols ou les mélanges desdits alcools. Dans le cas où le solvant est un amide, on peut faire appel notamment au diméthylformamide et au diméthylacétamide. Parmi les amines qui peuvent être utilisées comme solvant, on peut mettre en oeuvre par exemple l'amine, la diamine ou l'aminonitrile correspondant au nitrile ou au dinitrile que l'on hydrogène. La phase liquide comporte aussi en général une base minérale forte dérivant d'un métal alcalin ou alcalino-terreux.

Lorsque l'eau est présente avec un autre solvant, ledit solvant représente en poids de deux à quatre fois le poids de l'eau.

La phase catalytique solide est constituée en général d'un catalyseur à base de nickel de Raney et/ou de cobalt de Raney, comportant éventuellement, mais préférentiellement un élément dopant choisi parmi les éléments des groupes IVb, Vlb, Vllb et VIII de la classification périodique des éléments telle que publiée dans Handbook of Chemistry and Physics, 51st Edition (1970-1971).

Le catalyseur à base de nickel de Raney et/ou de cobalt de Raney utilisé dans le procédé peut donc comporter, outre le nickel ou le cobalt et les quantités résiduelles du métal éliminé de l'alliage d'origine lors de la préparation du catalyseur, c'est-à-dire généralement l'aluminium, un ou plusieurs autres éléments dopants, tels que par exemple le chrome, le titane, le molybdène, le tungstène, le fer, le zinc.

Parmi ces éléments dopants le chrome et/ou le fer et/ou le titane sont considérés comme les plus avantageux. Ces dopants représentent habituellement, en poids par poids de nickel, de 0 % à 15 % et de préférence de 0 % à 10 %.

La phase catalytique peut aussi être constituée par un métal, qui est généralement un métal du groupe VIII de la classification périodique des éléments tel que le ruthénium, le rhodium, le nickel ou le cobalt, déposé sur un support qui est généralement un oxyde métallique tel que les alumines, les silices, les aluminosilicates, le dioxyde de titane, l'oxyde de zirconium, l'oxyde de magnésium.

Dans les catalyseurs métalliques supportés, le métal représente généralement de 0,1 à 80 % du poids du support et préférentiellement de 0,5 à 50 %.

La phase solide représente généralement de 1% à 50 % en poids du poids de la phase liquide, sans que ces valeurs soient critiques.

La phase gazeuse est constituée essentiellement par l'hydrogène.

La filtration tangentielle peut être conduite à une température qui est avantageusement celle à laquelle est réalisée la réaction d'hydrogénation. Cette température est le plus souvent inférieure ou égale à 150°C, de préférence inférieure ou égale à 120°C et, plus préférentiellement encore, inférieure ou égale à 100°C.

Concrètement, cette température est habituellement comprise entre la température ambiante (20°C environ) et 100°C. On peut sans problème technique opérer à une température inférieure à 20°C, mais cela ne présente pas d'intérêt en raison d'une productivité plus faible de la réaction d'hydrogénation.

La différence de pression entre l'entrée et la sortie du filtre, nécessaire à la filtration peut être apportée en partie par la pression à laquelle est conduite la réaction d'hydrogénation. Mais il est nécessaire de créer une pression supérieure à la pression atmosphérique. Généralement cette pression est comprise entre 1 bar (0,10 MPa) et 20 bar (1 MPa) et de préférence entre 2 bar (0,5 MPa) et 10 bar (5 MPa). En pratique, la pression est créée par une pompe qui alimente à partir du réacteur le filtre à membrane.

Le flux alimentant le filtre à membrane dépend évidemment de la quantité de mélange réactionnel présent dans le réacteur ainsi que de la capacité du filtre. Il est également déterminé selon l'avancement de la réaction, de manière à ce que le perméat ou filtrat liquide, qui sera au moins partiellement récupéré et traité par ailleurs, contienne des quantités suffisantes du ou des produits visés par la réaction d'hydrogénation.

Le catalyseur filtré en présence d'hydrogène demeure actif et est recyclé dans la réaction d'hydrogénation.

L'exemple qui suit illustre l'invention.

### EXEMPLE

Un réacteur muni d'une agitation et de moyens de chauffage et de refoidissement, de moyens d'introduction de réactifs à sa partie supérieure et de soutirage à sa partie inférieure, contient environ 20 litres d'un mélange composé en poids de:
- 20,1 % d'adiponitrile
- 51,4 % d'aminocapronitrile
- 9,1 % d'hexaméthylène diamine
- 14,2 % d'eau
- 5,2 % de nickel de Raney ayant une granulométrie moyenne de 10 micromètres environ.

Cette solution est sous pression d'hydrogène de 2 bar et à une température de 55°C.

A la sortie de soutirage située à la partie inférieure du réacteur est placée une pompe volumétrique ayant un débit de 2 m³/h, suivie d'un débitmètre électromagnétique et d'un manomètre. La solution soutirée du réacteur alimente un filtre membranaire constitué d'un support en graphite et d'une couche active minérale en oxyde de zirconium ayant un seuil de coupure de 300 kD et un diamètre moyen de pores compris entre 25 et 50 nm (marque commerciale Carbosep M9 de la Société TECH-SEP).

Le flux d'alimentation du filtre est mis sous une pression d'hydrogène de 2 bar et la vitesse de circulation du mélange réactionnel sur la membrane est de 5m/s environ.

Le flux de perméat est de 67 kg/h.m². Le perméat est à la pression atmosphérique.

On n'observe pas de présence de nickel de Raney dans le perméat.

Le rétentat contenant le catalyseur est recyclé dans le réacteur.

Dans ces conditions, la concentration en catalyseur dans le rétentat est constante et égale à sa valeur initiale.

## Revendications

1. Procédé de filtration, caractérisé en ce que l'on réalise une filtration tangentielle sur un filtre à membrane d'au moins une partie d'un mélange réactionnel triphasique issue d'une réaction d'hydrogénation comportant une phase liquide dans laquelle se trouvent des fonctions nitrile, une phase gazeuse comprenant de l'hydrogène et une phase solide catalytique comprenant du nickel et/ou du cobalt de Raney ou un catalyseur métallique supporté, et en ce qu'on recycle le catalyseur filtré tout en soutirant au moins une partie du filtrat contenant les produits de la réaction.

2. Procédé selon la revendication 1, caractérisé en ce que le filtre à membrane utilisé pour la filtration tangentielle est constitué d'un support plan ou tubulaire et d'une membrane (ou couche active) minérale ou organique, de quelques micromètres d'épaisseur.

3. Procédé selon la revendication 2, caractérisé en ce que la couche active du filtre est en alpha-alumine, en oxyde de zirconium, en dioxyde de titane, en fibres de graphite et le support est en graphite, en alumine, en oxyde de zirconium, en dioxyde de titane.

4. Procédé selon l'une des revendications 2 ou 3, caractérisé en ce que la membrane présente un diamètre moyen de pores variant entre 1 nanomètre et un micromètre et de préférence un diamètre moyen de pores de 10 nanomètres à 100 nanomètres.

5. Procédé selon l'une des revendications 1 à 4, caractérisé en ce que la phase liquide du mélange réactionnel à filtrer comprend essentiellement au moins un composé à fonctions nitrile, tel que le dinitrile ou le nitrile de départ n'ayant pas été transformé, l'aminonitrile et/ou l'amine et/ou la diamine formés, un éventuel solvant.

6. Procédé selon la revendication 5, caractérisé en ce que le solvant est choisi parmi l'eau et/ou un amide et/ou un alcool et/ou une amine et/ou de l'ammoniac.

7. Procédé selon l'une des revendications 1 à 6, caractérisé en ce que la phase catalytique solide est constituée d'un catalyseur à base de nickel de Raney et/ou de cobalt de Raney, comportant préférentiellement un élément dopant choisi parmi les éléments des groupes IVb, Vlb, Vllb et VIII de la classification périodique des éléments telle que publiée dans Handbook of Chemistry and Physics, 51st Edition (1970-1971).

8. Procédé selon l'une des revendications 1 à 6, caractérisé en ce que la phase catalytique solide est constituée par un métal, qui est un métal du groupe VIII de la classification périodique des éléments tel que le ruthénium, le rhodium, le nickel ou le cobalt, déposé sur un support qui est un oxyde métallique tel que les alumines, les silices, les aluminosilicates, le dioxyde de titane, l'oxyde de zirconium, l'oxyde de magnésium.

9. Procédé selon l'une des revendications 1 à 8, caractérisé en ce que la filtration tangentielle est conduite à une température qui est inférieure ou égale à 150°C, de préférence inférieure ou égale à 120°C et, plus préférentiellement encore, inférieure ou égale à 100°C.

10. Procédé selon l'une des revendications 1 à 9, caractérisé en ce que la différence de pression entre l'entrée et la sortie du filtre est comprise entre 1 bar (0,10 MPa) et 20 bar (1 MPa) et de préférence entre 2 bar (0,5 MPa) et 10 bar (5 MPa).

## Claims

1. Filtration process, characterized in that a tangential filtration is carried out, on a membrane filter, of at least some of a three-phase reaction mixture resulting from a hydrogenation reaction comprising a liquid phase in which nitrile functions are found, a gaseous phase comprising hydrogen and a catalytic solid phase comprising Raney nickel and/or cobalt or a supported metal catalyst, and in that the filtered catalyst is recycled while at the same time at least some of the filtrate containing the reaction products is extracted.

2. Process according to Claim 1, characterized in that the membrane filter used for the tangential filtration consists of a flat or tubular support and of an inorganic or organic membrane (or active layer) which is a few micrometres in thickness.

3. Process according to Claim 2, characterized in that the active layer of the filter is made of α-alumina, zirconium oxide, titanium dioxide or graphite fibres and the support is made of graphite, alumina, zirconium oxide or titanium dioxide.

4. Process according to either of Claims 2 and 3, characterized in that the membrane has a mean pore diameter ranging between 1 nanometre and one micrometre and preferably a mean pore diameter of from 10 nanometres to 100 nanometres.

5. Process according to one of Claims 1 to 4, characterized in that the liquid phase of the reaction mixture to be filtered essentially comprises at least one compound containing nitrile functions, such as the unconverted starting dinitrile or nitrile, the aminonitrile and/or the amine and/or the diamine formed, and an optional solvent.

6. Process according to Claim 5, characterized in that the solvent is chosen from water and/or an amide and/or an alcohol and/or an amine and/or ammonia.

7. Process according to one of Claims 1 to 6, characterized in that the solid catalytic phase consists of a catalyst based on Raney nickel and/or Raney cobalt, preferably comprising a doping element chosen from the elements from Groups IVb, VIb, VIIb and VIII of the Periodic Table of the Elements as published in the Handbook of Chemistry and Physics, 51st edition (1970-1971).

8. Process according to one of Claims 1 to 6, characterized in that the solid catalytic phase consists of a metal, which is a metal from Group VIII of the Periodic Table of the Elements, such as ruthenium, rhodium, nickel or cobalt, deposited on a support which is a metal oxide, such as aluminas, silicas, aluminosilicates, titanium dioxide, zirconium oxide or magnesium oxide.

9. Process according to one of Claims 1 to 8, characterized in that the tangential filtration is carried out at a temperature which is less than or equal to 150°C, preferably less than or equal to 120°C and even more preferably less than or equal to 100°C.

10. Process according to one of Claims 1 to 9, characterized in that the pressure difference between the filter inlet and outlet is between 1 bar (0.10 MPa) and 20 bar (1 MPa) and preferably between 2 bar (0.5 MPa) and 10 bar (5 MPa).

## Patentansprüche

1. Filtrationsverfahren, dadurch gekennzeichnet, daß man eine tangentiale Filtration über einer Filtermembran von zumindest einem Teil eines dreiphasigen Reaktionsgemisches vornimmt, das hervorgegangen ist aus einer Hydrierungsreaktion, die eine flüssige Phase, in der sich Nitrilfunktionen befinden, eine Wasserstoff-umfassende Gasphase und eine katalytische feste Phase, umfassend Raney-Nickel und/oder Raney-Kobalt oder einen trägergestützten Metallkatalysator, beinhaltet und daß man den filtrierten Katalysator recyclisiert, wobei man stets zumindest einen Teil des die Reaktionsprodukte enthaltenden Filtrats abzieht.

2. Verfahren gemäß Anspruch 1, dadurch gekennzeichnet, daß der für die tangentiale Filtration verwendete Membranfilter aus einem ebenen oder röhrenförmigen Träger und einer mineralischen oder organischen Membran (oder aktiven Schicht) von einigen Mikrometern Dicke besteht.

3. Verfahren gemäß Anspruch 2, dadurch gekennzeichnet, daß die aktive Schicht des Filters aus alpha-Aluminiumoxid, aus Zirkonoxid, aus Titandioxid, aus Graphitfasern, besteht, und der Träger aus Graphit, aus Aluminiumoxid, aus Zirkoniumoxid, aus Titandioxid, besteht.

4. Verfahren gemäß einem der Ansprüche 2 oder 3, dadurch gekennzeichnet, daß die Membran einen durchschnittlichen Porendurchmesser, variierend zwischen einem Nanometer und einem Mikrometer, und vorzugsweise einen durchschnittlichen Porendurchmesser von 10 Nanometern bis 100 Nanometern, besitzt.

5. Verfahren gemäß einem der Ansprüche 1 bis 4, dadurch gekennzeichnet, daß die flüssige Phase des zu filtrierenden Reaktionsgemisches im wesentlichen zumindest eine Verbindung mit Nitrilfunktionen, wie Dinitril oder das nicht umgewandelte Ausgangsnitril, das gebildete Aminonitril und/oder Amin und/oder Diamin, gegebenenfalls ein Lösungsmittel umfaßt.

6. Verfahren gemäß Anspruch 5, dadurch gekennzeichnet, daß das Lösungsmittel unter Wasser und/oder einem Amid und/oder einem Alkohol und/oder einem Amin und/oder Ammoniak ausgewählt wird.

7. Verfahren gemäß einem der Ansprüche 1 bis 6, dadurch gekennzeichnet, daß die feste katalytische Phase aus einem Katalysator auf Basis von Raney-Nickel und/oder Raney-Kobalt, enthaltend vorzugsweise ein Dotierungselement, ausgewählt unter den Elementen der Gruppen IVb, VIb, VIIb und VIII des Periodensystems der Elemente, wie in dem Handbook of Chemistry and Physics, 51. Ausgabe (1970-1971) veröffentlicht, besteht.

8. Verfahren gemäß einem der Ansprüche 1 bis 6, dadurch gekennzeichnet, daß die feste katalytische Phase aus einem Metall, bei dem es sich um ein Metall der Gruppe VIII des Periodensystems der Elemente, wie um Ruthenium, Rhodium, Nickel oder Kobalt handelt, abgeschieden auf einem Träger, bei dem es sich um ein Metalloxid, wie die Aluminiumoxide, die Siliciumdioxide, die Alumosilikate, Titandioxid, Zirkonoxid, Magnesiumoxid, handelt, besteht.

9. Verfahren gemäß einem der Ansprüche 1 bis 8, dadurch gekennzeichnet, daß die tangentiale Filtration bei einer Temperatur durchgeführt wird, die niedriger ist als oder gleich 150°C, vorzugsweise niedriger als oder gleich 120°C, insbesondere niedriger als oder gleich 100°C.

10. Verfahren gemäß einem der Ansprüche 1 bis 9, dadurch gekennzeichnet, daß der Druckunterschied zwischen dem Eintritt und dem Austritt des Filters zwischen 1 bar (0,10 MPa) und 20 bar (1 MPa) und vorzugsweise zwischen 2 bar (0,5 MPa) und 10 bar (5 MPa) beträgt.
